# EUROPEAN PATENT SPECIFICATION

(11) **EP 4 293 792 B1**
(45) Date of publication and mention of the grant of the patent: **27.08.2025**
(21) Application number: 22864855.6
(22) Date of filing: 11.07.2022
(51) Int. Cl.: H01M 10/42, H01M 10/48, G01N 33/00, H01M 10/0525

(54) **BATTERY MANAGEMENT APPARATUS AND OPERATING METHOD THEREOF**
BATTERIEVERWALTUNGSVORRICHTUNG UND BETRIEBSVERFAHREN DAFÜR
DISPOSITIF DE GESTION DE BATTERIE ET SON PROCÉDÉ DE FONCTIONNEMENT

(30) Priority: 30.08.2021 KR 20210115108
(43) Date of publication of application: 20.12.2023
(73) Proprietor: LG Energy Solution, Ltd., Seoul 07335 (KR)
(72) Inventor: KIM, Won Hee, Daejeon 34122 (KR)
(74) Representative: Plasseraud IP
(86) International application number: PCT/KR2022/010030
(87) International publication number: WO 2023/033346

(56) References cited:
- JP-A- 2010 151 756
- JP-A- 2010 151 756
- JP-A- 2014 514 909
- KR-A- 20140 015 702
- KR-A- 20140 015 702
- KR-A- 20150 011 426
- US-A1- 2018 102 572

## Description

### [TECHNICAL FIELD]

### CROSS-REFERENCE TO RELATED APPLICATION

This application claims priority to and the benefit of Korean Patent Application No. 10-2021-0115108 filed in the Korean Intellectual Property Office on August 30, 2021.

### TECHNICAL FIELD

Embodiments disclosed herein relate to a battery management apparatus and an operating method thereof.

### [BACKGROUND ART]

Recently, research and development of secondary batteries have been actively performed. Herein, the secondary batteries, which are chargeable/dischargeable batteries, may include all of conventional nickel (Ni)/cadmium (Cd) batteries, Ni/metal hydride (MH) batteries, etc., and recent lithium-ion batteries. Among the secondary batteries, a lithium-ion battery has a much higher energy density than those of the conventional Ni/Cd batteries, Ni/MH batteries, etc. Moreover, the lithium-ion battery may be manufactured to be small and lightweight, such that the lithium-ion battery has been used as a power source of mobile devices, and recently, a use range thereof has been extended to power sources for electric vehicles, attracting attention as next-generation energy storage media.

Secondary batteries are generally used as battery racks including a battery module where a plurality of battery cells are connected to one another in series and/or in parallel. The battery rack may be managed and controlled by a battery management system in terms of a state and an operation. An energy storage system (ESS) including the battery rack mainly obtains key data through a personal computer (PC)-based battery management system and various sensors provided on the site.

To stably operate the ESS, diagnosis through a gas sensor is required. To use the gas sensor, a time for gathering base line information is required to obtain the base line information, and in case of an error occurring in the gas sensor, a recovery time may be delayed.

Document KR20140015702A discloses a battery management system of the related art.

### [DISCLOSURE]

### [TECHNICAL PROBLEM]

Embodiments disclosed herein aim to provide a battery management apparatus and an operating method thereof to reduce a recovery time for allowing a gas sensor to normally operate, in case of a failure error occurring in the gas sensor.

Technical problems of the embodiments disclosed herein are not limited to the above-described technical problems, and other unmentioned technical problems would be clearly understood by one of ordinary skill in the art from the following description.

### [Technical Solution]

A battery management apparatus according to an embodiment disclosed herein is defined in the appended claims.
An operating method of a battery management apparatus is also provided.

### [ADVANTAGEOUS EFFECTS]

A battery management apparatus and an operating method thereof according to an embodiment disclosed herein may reduce a time for obtaining base line information by obtaining the base line information from a higher-level battery management system (BMS) in case of an error occurring in a gas sensor.

The battery management apparatus and the operating method thereof according to an embodiment disclosed herein may set identification information of each of a plurality of gas sensors, thereby accurately delivering the base line information obtained from the higher-level BMS to each gas sensor.

The battery management apparatus and the operating method thereof according to an embodiment disclosed herein may set identification information for each of a plurality of gas sensors through a serially connected power supply line for identification information allocation, thereby setting the same identification information in spite of hardware replacement of the gas sensor.

The battery management apparatus and the operating method thereof according to an embodiment disclosed herein may stably operate a battery using a gas sensor by reducing a recovery time for allowing the gas sensor to operate even when an error frequently occurs in the plurality of gas sensors.

Moreover, various effects recognized directly or indirectly from the disclosure may be provided.

### [DESCRIPTION OF DRAWINGS]

FIG. 1 shows a battery management apparatus and a plurality of gas sensors, according to an embodiment disclosed herein.
FIG. 2 is a block diagram of a battery management apparatus according to an embodiment disclosed herein.
FIG. 3 shows a system including a battery management apparatus, according to an embodiment disclosed herein.
FIG. 4 is a flowchart of an operating method of a battery management apparatus according to an embodiment disclosed herein.
FIGS. 5 to 7 are flowcharts of an operating method of a battery management apparatus, according to an embodiment disclosed herein.
FIG. 8 is a block diagram showing a hardware configuration of a computing system for performing a control method of a battery management apparatus, according to an embodiment disclosed herein.

### [MODE FOR INVENTION]

Hereinafter, embodiments disclosed in this document will be described in detail with reference to the exemplary drawings. In adding reference numerals to components of each drawing, it should be noted that the same components are given the same reference numerals even though they are indicated in different drawings. In addition, in describing the embodiments disclosed in this document, when it is determined that a detailed description of a related known configuration or function interferes with the understanding of an embodiment disclosed in this document, the detailed description thereof will be omitted.

To describe a component of an embodiment disclosed herein, terms such as first, second, A, B, (a), (b), etc., may be used. These terms are used merely for distinguishing one component from another component and do not limit the component to the essence, sequence, order, etc., of the component. The terms used herein, including technical and scientific terms, have the same meanings as terms that are generally understood by those skilled in the art, as long as the terms are not differently defined. Generally, the terms defined in a generally used dictionary should be interpreted as having the same meanings as the contextual meanings of the relevant technology and should not be interpreted as having ideal or exaggerated meanings unless they are clearly defined in the present application.

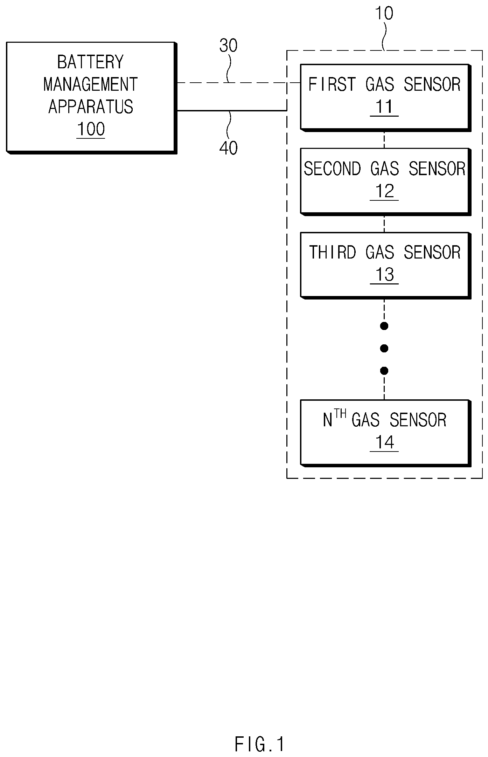

FIG. 1 shows a battery management apparatus and a plurality of gas sensors, according to an embodiment disclosed herein.

Although the following description will be made of an example where a sensor according to the present disclosure is implemented as a gas sensor, the sensor may be implemented as various sensors, such as a temperature sensor, a humidity sensor, etc., capable of transmitting base line information to a higher-level battery management system (BMS) depending on an embodiment.

Referring to FIG. 1, a plurality of gas sensors 10 may include a first gas sensor 11 and a second gas sensor 12 to an n^{th} gas sensor 14. While it is illustrated in FIG. 1 that the number of gas sensors included in the plurality of gas sensors 10 is 4, the plurality of gas sensors 10 may include n gas sensors (n is a natural number greater than or equal to 2).

The battery management apparatus 100 may be connected to the plurality of gas sensors 10 through a first line 30 and a second line 40. For example, the first line 30 may be a power supply line for identification information allocation. In another example, the second line 40 may be a line for power supply to the plurality of gas sensors 10.

The battery management apparatus 100 may supply power to the plurality of gas sensors 10 through the second line 40. For example, the battery management apparatus 100 may control power of a battery to be supplied to the plurality of gas sensors 10 through the second line 40. In another example, the battery management apparatus 100 may control independent power only for the plurality of gas sensors 10 to be supplied to the plurality of gas sensors 10 through the second line 40. In an embodiment, the battery management apparatus 100 may determine whether to supply power to the plurality of gas sensors 10 by controlling an operation of a relay (not shown) installed in the second line 40. In an embodiment, the second line 40 may be serially and/or in parallel connected to the plurality of gas sensors 10 which may receive power through the second line 40.

In an embodiment, the battery management apparatus 100 may stop supplying power to the plurality of gas sensors 10 when determining that an error occurs in at least any one of the plurality of gas sensors 10. For example, when an error occurs in at least any one gas sensor, it may be a case where the gas sensor fails to operate normally or a failure occurs in the gas sensor, such that the battery management apparatus 100 may stop supplying power to the plurality of gas sensors 100. In this case, the battery management apparatus 100 may inform a user that an error occurs in the gas sensor, and the user may recognize the error occurring in the gas sensor, and take an appropriate action (e.g., hardware replacement of the gas sensor). When an error does not occur in the plurality of gas sensors 10 or the error occurring in the plurality of gas sensors 10 is solved, the battery management apparatus 100 may control power to be supplied to the plurality of gas sensors 10 through the second line 40.

Each of the plurality of gas sensors 10 may be serially connected to a power supply line for identification information allocation. For example, the battery management apparatus 100 may be connected to the first gas sensor 11 among the plurality of gas sensors 10 through the first line 30, and the first gas sensor 11 may be serially connected to the second gas sensor 12 through the first line 30. The second gas sensor 12 may be serially connected to the third gas sensor 130 through the first line 30, and the plurality of gas sensors 10 may be serially connected through the first line 30. In an embodiment, the first line 30 may be a power supply line for identification information allocation.

The battery management apparatus 100 may set identification information for each of the plurality of gas sensors 10 through the power supply line for identification information allocation (the first line 30). For example, the battery management apparatus 100 may set identification information when power for identification information allocation is applied to each of the plurality of gas sensors 10.

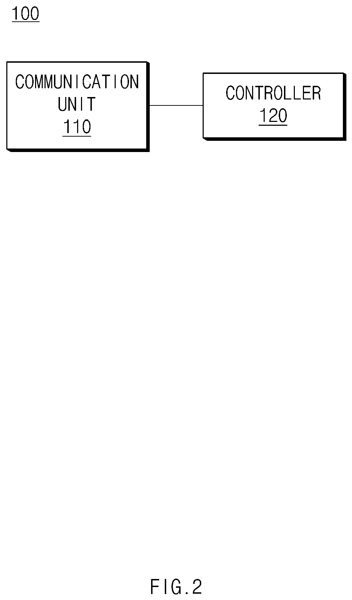

Hereinbelow, referring to FIGS. 1 and 2, an operation of the battery management apparatus 100 will be described.

FIG. 2 is a block diagram of a battery management apparatus according to an embodiment disclosed herein.

Referring to FIG. 2, the battery management apparatus 100 according to an embodiment disclosed herein may include a communication unit 110 and a controller 120. The battery management apparatus 100 may be substantially the same as the battery management apparatus 100 of FIG. 1.

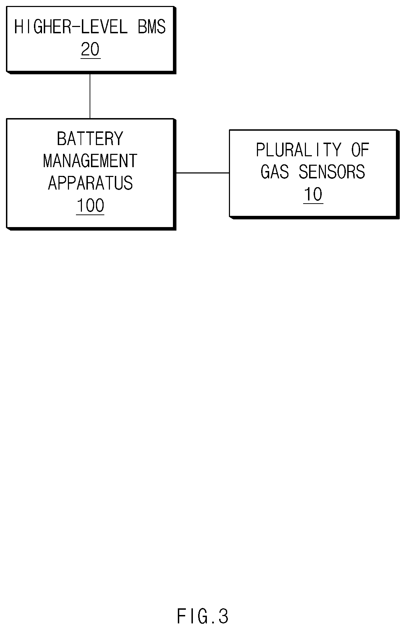

The communication unit 110 may communicate with the plurality of gas sensors 10 (see FIG. 1). The communication unit 110 may communicate with a higher-level BMS 20 (see FIG. 3). In an embodiment, the communication unit 110 may include a wireless communication circuit (e.g., a cellular communication circuit, a short-range wireless communication circuit, or a global navigation satellite system (GNSS) communication circuit) or a wired communication circuit (e.g., a local area network (LAN) communication circuit or a power-line communication circuit), and may communicate with the external electronic device by using a corresponding communication circuit, via a short-range communication network such as Bluetooth, wireless-fidelity (Wi-Fi) direct, or infrared data association (IrDA) or a long-range communication network such as a cellular network, the Internet, or a computer network. The above-enumerated various types of communication unit 110 may be implemented in a single chip or individually in separate chips.

In an embodiment, the battery management apparatus 100 may control whether to supply power to the plurality of gas sensors 10. For example, the controller 120 may control power supply to the plurality of gas sensors 10 to be stopped when determining that an error occurs in at least any one of the plurality of gas sensors 10.

The controller 120 may set identification information of each of the plurality of gas sensors 10. For example, the controller 120 may set an identification (ID) of each of the plurality of gas sensors 10. In another example, the controller 120 may set identification information based on position information of each of the plurality of gas sensors 10. In this case, the position information of each of the plurality of gas sensors 10 may indicate an order of a corresponding gas sensor with respect to a gas sensor connected to the controller 120. In an embodiment, the controller 120 may be a micro controller unit (MCU).

The controller 120 may apply power for identification information allocation to the first gas sensor connected to the power supply line 30 for identification information allocation among the plurality of gas sensors 10. For example, the controller 120 may apply power for identification information allocation to the first gas sensor 11 through the power supply line 30 for identification information allocation. The controller 120 may set identification information for the first gas sensor 11 when power for identification information allocation is applied to the first gas sensor 11. For example, the controller 120 may set identification information '0001' for the first gas sensor 11.

In an embodiment, the controller 120 may supply power for identification information allocation to the first gas sensor 11 by controlling a relay (not shown) existing in the power supply line 30 for identification information allocation to be shorted. In this case, the other gas sensors than the first gas sensor 11 among the plurality of gas sensors 10 may not receive power for identification information allocation, and identification information only for the first gas sensor 11 may be set. For example, a relay (not shown) may exist in the power supply line 30 for identification information allocation to which the first gas sensor 11 and the second gas sensor 12 are serially connected, and the relay (not shown) is opened, such that the second gas sensor 12 may not receive power for identification information allocation.

The controller 120 may set identification information when each of the plurality of gas sensors 10 is sequentially provided with power for identification information allocation from another gas sensor through the power supply line 30 for identification information allocation. For example, the controller 120 may sequentially set identification information for a gas sensor that receives power for identification information allocation.

In an embodiment, when the first gas sensor 11 is set with identification information from the controller 120, the first gas sensor 11 may supply the power for identification information allocation to the second gas sensor 12 serially connected to the power supply line 30 for identification information allocation. For example, the controller 11 may supply the power for identification information allocation to the second gas sensor 12 by shorting a relay (not shown) existing in the power supply line 30 for identification information allocation between the first gas sensor 11 and the second gas sensor 12. In this case, the controller 120 may set the identification information for the second gas sensor 12 because the power for identification information allocation is supplied to the second gas sensor 12. For example, the controller 120 may set identification information '0002' for the second gas sensor 12.

In an embodiment, when the identification information is set for the second gas sensor 12, the second gas sensor 12 may supply the power for identification information allocation to the third gas sensor 13 serially connected to the power supply line 30 for identification information allocation and the controller 120 may set the identification information for the third gas sensor 13. Thereafter, the controller 120 may sequentially set identification information for the plurality of gas sensors 10, and set identification information up to the n^{th} gas sensor 14.

The battery management apparatus 100 according to an embodiment disclosed herein may set identification information for the plurality of gas sensors 10. The battery management apparatus 100 sets identification information according to the order of a gas sensor serially connected to a power supply line for identification information allocation, such that in spite of hardware replacement due to an error in some gas sensors, the same identification information as that for the gas sensor before replacement may be set as long as the gas sensor is installed at the same position. That is, when the plurality of gas sensors 10 are powered off and then on due to an error in some of the plurality of gas sensors 10, the battery management apparatus 100 may set identification information of each of the plurality of gas sensors 10 identically to that before occurrence of the error.

Hereinbelow, referring to FIGS. 2 and 3, an operation of the battery management apparatus 100 will be described.

FIG. 3 shows a system including a battery management apparatus, according to an embodiment disclosed herein. In an embodiment, the battery management apparatus 100 may be substantially the same as the battery management apparatus 100 of FIGS. 1 and 2, and the plurality of gas sensors 10 may be substantially the same as the plurality of gas sensors 10 of FIG. 1.

Referring to FIG. 3, the battery management apparatus 100 may communicate with the plurality of gas sensors 10 and the higher-level BMS 20. For example, the communication unit 110 included in the battery management apparatus 100 may communicate with the plurality of gas sensors 10 and the higher-level BMS 20.

The controller 120 included in the battery management apparatus 100 may obtain base line information of each of the plurality of gas sensors 10 based on identification information. For example, the controller 120 may obtain base line information of each of the plurality of gas sensors 10 from the higher-level BMS 20 based on identification information of each of the plurality of gas sensors 10.

In an embodiment, the higher-level BMS 20 may obtain the base line information of each of the plurality of gas sensors 10 from the battery management apparatus 100 based on identification information of each of the plurality of gas sensors 10, and store the obtained base line information based on the identification information of each of the plurality of gas sensors 10. The higher-level BMS 20 may deliver base line information corresponding to identification information of the plurality of gas sensors 10 to the battery management apparatus 100, when there is a request for the base line information of the plurality of gas sensors 10 from the battery management apparatus 100.

When the plurality of gas sensors are powered off and then on due to an error occurring in the plurality of gas sensors, the controller 120 may obtain base line information of each of the plurality of gas sensors 10 from the higher-level BMS. For example, when an error occurs in the plurality of gas sensors, the controller 120 may control the plurality of gas sensors 10 to be powered off, and when the plurality of gas sensors 10 are powered on after the error occurring in the plurality of gas 10 is solved, the controller 120 may obtain the base line information from the higher-level BMS 20.

When the controller 120 obtains the base line information of the plurality of gas sensors 10 from the higher-level BMS 20 based on the identification information of each of the plurality of gas sensors 10, the controller 120 may deliver the obtained base line information to the plurality of gas sensors 10. For example, the controller 120 may deliver the base line information to each of the plurality of gas sensors 10 based on the identification information of each of the plurality of gas sensors 10. Each of the plurality of gas sensors 10 may perform diagnosis on a battery based on the delivered base line information.

In an embodiment, the controller 120 may control the plurality of gas sensors 10 to obtain the base line information when failing to obtain the base line information of the plurality of gas sensors 10 from the higher-level BMS 20. For example, when the base line information of the plurality of gas sensors 10 does not exist in the higher-level BMS 20, the controller 120 may fail to obtain the base line information and obtain base line information measured for a preset time by the plurality of gas sensors 10. In another example, the preset time may be 24 hours.

When the controller 120 obtains the base line information measured by each of the plurality of gas sensors 10 from the plurality of gas sensors 10, the controller 120 may deliver the measured base line information to the higher-level BMS 20. For example, when the plurality of gas sensors 10 are powered off and then on due to an error occurring in the plurality of gas sensors 10 after the base line information is delivered to the higher-level BMS 20, the controller 120 obtains again the base line information delivered to the higher-level BMS 20 and delivers the same to the plurality of gas sensors 10, thus reducing a recovery time of the plurality of gas sensors 10.

In an embodiment, when an error does not occur in the plurality of gas sensors 10, the controller 120 may control the plurality of gas sensors 10 to measure the base line information for the preset time. For example, the controller 120 may control the plurality of gas sensors 10 to continuously measure the base line information simultaneously with diagnosing abnormality of the battery, and continuously update the base line information. In another example, the controller 120 may discard the measured base line information, including a time in which abnormality of the battery occurs, when the plurality of gas sensors 10 diagnose the abnormality of the battery based on the preset base line information.

The battery management apparatus 100 according to an embodiment disclosed herein may set identification information for each of the plurality of gas sensors 10, and obtain base line information of each of the plurality of gas sensors 10 from the higher-level BMS 20 based on the set identification information, thus effectively recovering the plurality of gas sensors 10 in case of the error occurring in the plurality of gas sensors 10. The battery management apparatus 100 may use previously measured base line information of the plurality of gas sensors 10 because the same identification information is set for a gas sensor existing in the same order. Moreover, when no error occurs in the plurality of gas sensors 10, the battery management apparatus 100 may control abnormality of the battery to be diagnosed based on the base line information and deliver a diagnosis result to the higher-level BMS 20, thus performing failure (abnormality) diagnosis of the battery through the gas sensor.

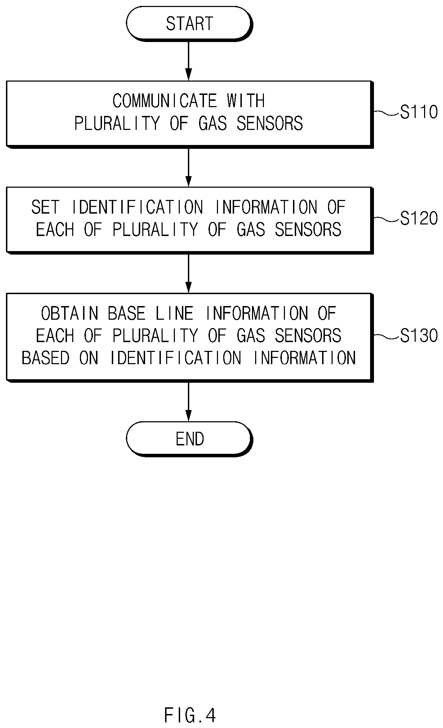

FIG. 4 is a flowchart of an operating method of a battery management apparatus according to an embodiment disclosed herein.

Referring to FIG. 4, an operating method of the battery management apparatus 100 according to an embodiment disclosed herein may include operation S 110 of communicating with a plurality of gas sensors, operation S120 of setting identification information of each of the plurality of gas sensors, and operation S130 of obtaining base line information of each of the plurality of gas sensors based on the identification information.

In operation S110 of communicating with the plurality of gas sensors, the communication unit 110 may communicate with the plurality of gas sensors.

In operation S120 of communicating with the plurality of gas sensors, the controller 120 may set the identification information of each of the plurality of gas sensors. For example, the controller 120 may control identical identification information to be set at all times for each of the plurality of gas sensors, by sequentially setting the identification information of each of the plurality of gas sensors. In another example, the controller 120 may set the identification information when power for identification information allocation is applied to each of the plurality of gas sensors.

In operation S130 of obtaining the base line information of each of the plurality of gas sensors based on the identification information, the controller 120 may obtain the base line information of each of the plurality of gas sensors based on the identification information. For example, the controller 120 may obtain the base line information of each of the plurality of gas sensors from the higher-level BMS based on the identification information of each of the plurality of gas sensors.

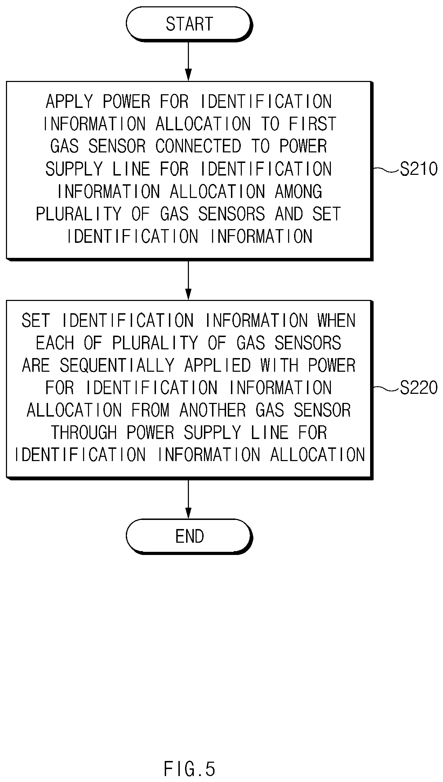

FIGS. 5 to 7 are flowcharts of an operating method of a battery management apparatus, according to an embodiment disclosed herein.

Referring to FIG. 5, an operating method of the battery management apparatus 100 according to an embodiment disclosed herein may include operation S210 of applying power for identification information allocation to the first gas sensor connected to a power supply line for identification information allocation among the plurality of gas sensors and setting identification information, and operation S220 of setting the identification information when each of the plurality of gas sensors is sequentially provided with the power for identification information allocation through the power supply line for identification information allocation from another gas sensor. For example, operations S210 and S220 may include operation S120 of FIG. 4.

In operation S210 of applying the power for identification information allocation to the first gas sensor connected to the power supply line for identification information allocation among the plurality of gas sensors and setting the identification information, the controller 120 may apply the power for identification information allocation to the first gas sensor connected to the power supply line for identification information allocation among the plurality of gas sensors. For example, the power supply line for identification information allocation may be different from a line for power supply to the plurality of gas sensors. In another example, the controller 120 may set the identification information for the first gas sensor when power for identification information allocation is applied to the first gas sensor. In another example, the power for identification information allocation is applied only to the first gas sensor, the controller 120 may not allocate identification information to the other gas sensors.

In operation S220 of setting the identification information when each of the plurality of gas sensors is sequentially provided with the power for identification information allocation through the power supply line for identification information allocation from another gas sensor, the controller 120 may set the identification information for each gas sensor when each of the plurality of gas sensors is sequentially provided with the power for identification information allocation from another gas sensor through the power supply line for identification information allocation. For example, the first gas sensor may supply the power for identification information allocation to the second gas sensor serially connected through the power supply line for identification information allocation, and the controller 120 may allocate the identification information when the power for identification information allocation is supplied to the second gas sensor. In another example, each of the plurality of gas sensors may supply the power for identification information allocation to the next gas sensor, and the controller 120 may sequentially set the identification information for a gas sensor provided with the power for identification information allocation.

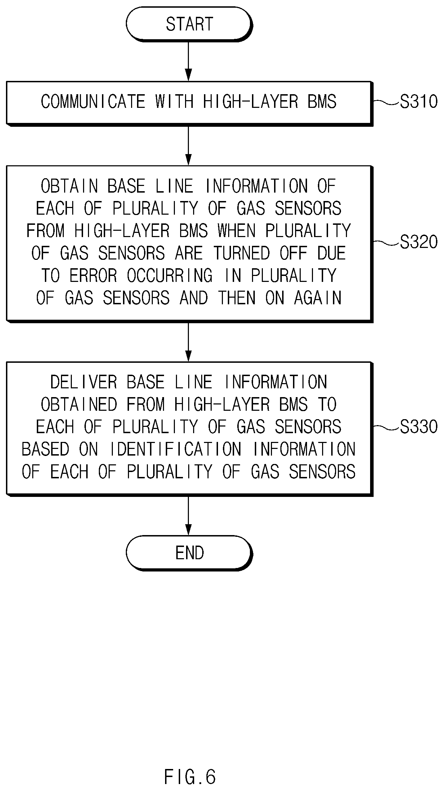

Referring to FIG. 6, the operating method of the battery management apparatus 100 according to an embodiment disclosed herein may further include operation S310 of communicating with the higher-level BMS, operation S320 of obtaining the base line information of each of the plurality of gas sensors from the higher-level BMS when the plurality of gas sensors are powered off and then on due to an error occurring in the plurality of gas sensors, and operation S330 of delivering the base line information obtained from the higher-level BMS to each of the plurality of gas sensors based on the identification information of each of the plurality of gas sensors.

In operation S310 of communicating with the higher-level BMS, the communication unit 110 may communicate with the higher-level BMS.

In operation S320 of obtaining the base line information of each of the plurality of gas sensors from the higher-level BMS when the plurality of gas sensors are powered off and then on due to the error occurring in the plurality of gas sensors, the controller 120 may control the plurality of gas sensors to be powered off when an error occurs in the plurality of gas sensors. The controller 120 may control the plurality of gas sensors to be powered on again when the error in the plurality of gas sensors is solved. When the plurality of gas sensors are powered on, the controller 120 may obtain the base line information of each of the plurality of gas sensors from the higher-level BMS. For example, the controller 120 may obtain the base line information of each of the plurality of gas sensors based on the identification information of each of the plurality of gas sensors. In an embodiment, when the base line information of each of the plurality of gas sensors does not exist in the higher-level BMS, the controller 120 may control the plurality of gas sensors to measure again the base line information of each of the plurality of gas sensors.

In operation S330 of delivering the base line information obtained from the higher-level BMS to each of the plurality of gas sensors based on the identification information of each of the plurality of gas sensors, the controller 120 may deliver the base line information to each of the plurality of gas sensors based on the identification information of each of the plurality of gas sensors when there is the base line information obtained from the higher-level BMS. For example, the identification information of each of the plurality of gas sensors is set according to an order in which the plurality of gas sensors are serially connected to the power for identification information allocation, such that the controller 120 may quickly recover an error occurring in a gas sensor by delivering the base line information of each of the plurality of gas sensors based on the identification information of each of the plurality of gas sensors.

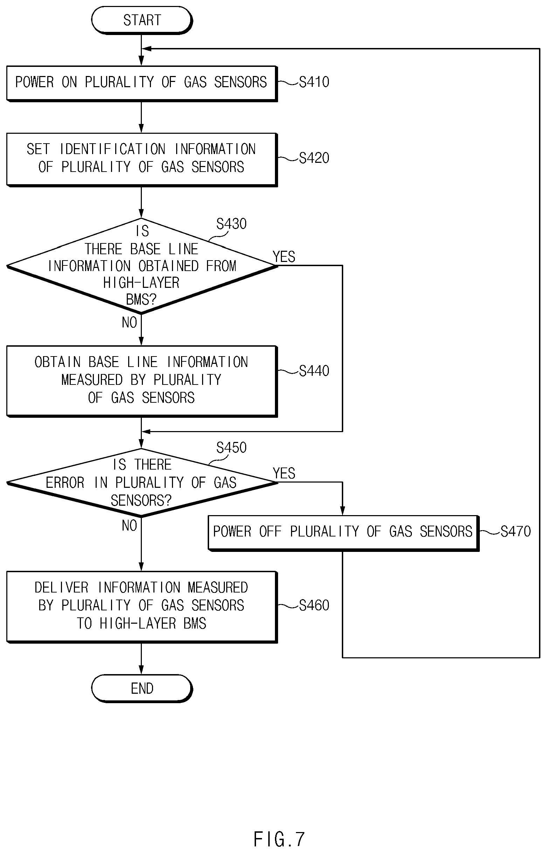

Referring to FIG. 7, the operating method of the battery management apparatus 100 according to an embodiment disclosed herein may include operation S410 of controlling the plurality of gas sensors to be powered on, operation S420 of setting the identification information of the plurality of gas sensors, operation S430 of determining whether there is the base line information obtained from the higher-level BMS, operation S440 of obtaining the base line information measured by the plurality of gas sensors, operation S450 of determining whether an error occurs in the plurality of gas sensors, operation S460 of delivering information measured by the plurality of gas sensors to the higher-level BMS, and operation S470 of controlling the plurality of gas sensors to be powered off.

In operation S410 of controlling the plurality of gas sensors to be powered on, the controller 120 may control the plurality of gas sensors to be powered on. For example, when the plurality of gas sensors are powered off due to the error occurring in the plurality of gas sensors and the plurality of gas sensors need to be powered on due to replacement or repair of the gas sensor having the error, the controller 120 may control the plurality of gas sensors to be powered on.

In operation S420 of setting the identification information of the plurality of gas sensors, the controller 120 may set the identification information of the plurality of gas sensors. For example, the battery management apparatus 100 and the plurality of gas sensors may be serially connected to the power supply line for identification information allocation, and the controller 120 may set the identification information for the gas sensor supplied with the power for identification information allocation through the measured power supply line for identification information allocation.

In operation S430 of determining whether there is the base line information obtained from the higher-level BMS, the controller 120 may request the base line information of the plurality of gas sensors to the higher-level BMS and obtain the base line information from the higher-level BMS. The controller 120 may determine whether the base line information is obtained from the higher-level BMS and perform operation S450 when the base line information is obtained from the higher-level BMS, and perform operation S440 when the base line information is not obtained. In an embodiment, when the base line information is obtained from the higher-level BMS, the controller 120 may deliver the obtained base line information to the plurality of gas sensors based on the identification information of each of the plurality of gas sensors.

In operation S440 of obtaining the base line information measured by the plurality of gas sensors, the controller 120 may obtain the base line information measured by the plurality of gas sensors because there is no base line information obtained from the higher-level BMS. For example, the plurality of gas sensors may transmit the base line information measured for a preset time to the controller 120 which may store the base line information measured by each of the plurality of gas sensors based on the identification information of each of the plurality of gas sensors or transmit the base line information to the higher-level BMS. In an embodiment, the preset time may be 24 hours. In an embodiment, operation S440 may not be performed when the base line information is previously stored in the higher-level BMS.

In operation S450 of determining whether there is an error in the plurality of gas sensors, the controller 120 may continuously determine whether there is an error during an operation of the plurality of gas sensors. For example, the controller 120 may perform operation S460 when determining that there is no error in the plurality of gas sensors. In another example, the controller 120 may perform operation S470 when there is an error in at least any one of the plurality of gas sensors.

In operation S460 of delivering the information measured by the plurality of gas sensors to the higher-level BMS, the controller 120 may deliver the information measured by the plurality of gas sensors to the higher-level BMS when no error occurs in the plurality of gas sensors. For example, the plurality of gas sensors may diagnose abnormality of the battery based on the base line information or measure state information of the battery and transmit the diagnosed or measured information to the controller 120. In this case, the controller 120 may deliver the information obtained from the plurality of gas sensors to the higher-level BMS which may diagnose abnormality of the battery based on the information measured by the plurality of gas sensors.

In operation S470 of controlling the plurality of gas sensors to be powered off, the controller 120 may control all of the plurality of gas sensors to be powered off when an error occurs in at least any one of the plurality of gas sensors. For example, when all of the plurality of gas sensors are powered off, the controller 120 may obtain again the base line information stored in the higher-level BMS from the higher-level BMS because the base line information of the plurality of gas sensors is lost. In an embodiment, the controller 120 may perform operation S410 when the error occurring in the plurality of gas sensors is solved.

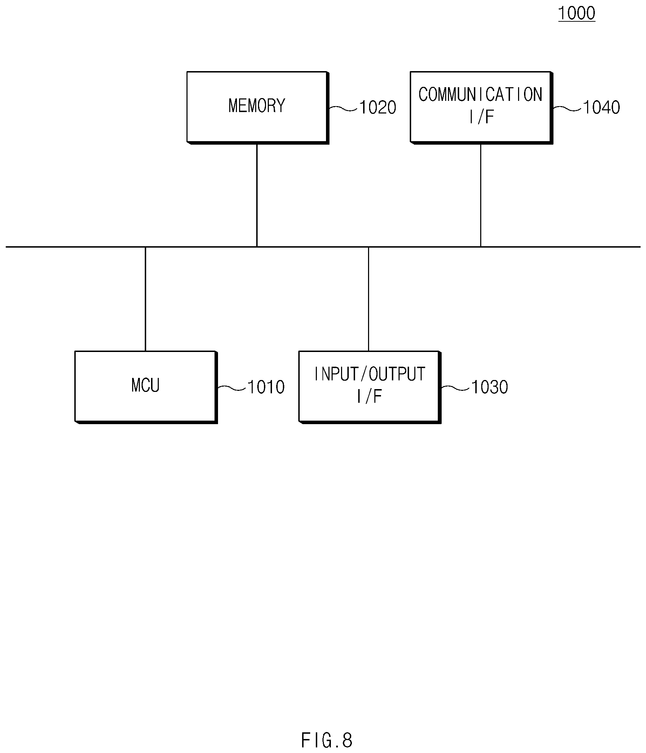

FIG. 8 is a block diagram showing a hardware configuration of a computing system for performing a control method of a battery management apparatus, according to an embodiment disclosed herein.

Referring to FIG. 8, a computing system 1000 according to an embodiment disclosed herein may include an MCU 1010, a memory 1020, an input/output interface (I/F) 1030, and a communication I/F 1040.

The MCU 1010 may be a processor that executes various programs (e.g., a battery cell voltage measurement program, a switching control program, etc.) stored in the memory 1020, processes various data including voltage, internal resistance, etc., of a battery cell through these programs, and performs the above-described functions of the battery management apparatus 100 shown in FIG. 2.

The memory 1020 may store various programs regarding voltage measurement, switching control, etc., of the battery cell. Moreover, the memory 1020 may store various data such as a voltage, an internal resistance, etc., of the battery cell.

The memory 1020 may be provided in plural, depending on a need. The memory 1020 may be volatile memory or non-volatile memory. For the memory 1020 as the volatile memory, random access memory (RAM), dynamic RAM (DRAM), static RAM (SRAM), etc., may be used. For the memory 1020 as the nonvolatile memory, read only memory (ROM), programmable ROM (PROM), electrically alterable ROM (EAROM), erasable PROM (EPROM), electrically erasable PROM (EEPROM), flash memory, etc., may be used. The above-listed examples of the memory 1020 are merely examples and are not limited thereto.

The input/output interface (I/F) 1030 may provide an interface for transmitting and receiving data by connecting an input device (not shown) such as a keyboard, a mouse, a touch panel, etc., and an output device such as a display (not shown), etc., to the MCU 1010.

The communication I/F 1040, which is a component capable of transmitting and receiving various data to and from a server, may be various devices capable of supporting wired or wireless communication. For example, a program for voltage measurement and switching control for a battery cell, various data, etc., may be transmitted and received to and from a separately provided external server through the communication I/F 1040.

As such, a computer program according to an embodiment disclosed herein may be recorded in the memory 1020 and processed by the MCU 1010, thus being implemented as a module that performs functions shown in FIG. 2.

The above description is merely illustrative of the technical idea of the present disclosure, and various modifications and variations will be possible without departing from the essential characteristics of embodiments of the present disclosure by those of ordinary skill in the art to which the embodiments disclosed herein pertains.

## Claims

1. A battery management apparatus (100) comprising:
a communication unit (110) configured to communicate with a plurality of sensors (10); and
a controller (120) configured to set identification information of each of the plurality of sensors (10) and obtain base line information of each of the plurality of sensors (10) based on the identification information
**characterized in that** the plurality of sensors (10) comprises a plurality of gas sensors, and
the communication unit (110) is further configured to communicate with a higher-level battery management system, BMS, and
the controller (120) is further configured to obtain base line information of each of the plurality of gas sensors (10) from the higher-level battery management system, BMS, when the plurality of gas sensors (10) are powered off and then on due to an error occurring in the plurality of gas sensors (10).

2. The battery management apparatus of claim 1, wherein the plurality of sensors (10) comprises a plurality of gas sensors (10), and
each of the plurality of gas sensors (10) are serially connected to a power supply line (30) for identification information allocation.

3. The battery management apparatus of claim 2, wherein the controller (120) is further configured to set the identification information when power for identification information allocation is applied to each of the plurality of gas sensors (10).

4. The battery management apparatus of claim 2, wherein the controller (120) is further configured to apply power for identification information allocation to a first gas sensor (11) connected to the power supply line for identification information allocation among the plurality of gas sensors (10) and set the identification information.

5. The battery management apparatus of claim 4, wherein the controller (120) is further configured to set the identification information when each of the plurality of gas sensors (10) is sequentially applied with the power for identification information allocation from another gas sensor (10) through the power supply line (30) for identification information allocation.

6. The battery management apparatus of claim 1, wherein the controller (120) is further configured to deliver the information obtained from the higher-level battery management system, BMS, to each of the plurality of gas sensors (10) based on the identification information of each of the plurality of gas sensors.

7. The battery management apparatus of claim 1, wherein the controller (120) is further configured to obtain information measured for a preset time by the plurality of sensors (10) when an error does not occur in the plurality of sensors (10).

8. The battery management apparatus of claim 7, wherein the communication unit (110) is further configured to communicate with the higher-level battery management system, BMS, and
the controller (120) is further configured to deliver the information measured from the plurality of sensors (10) to the higher-level battery management system, BMS

9. The battery management apparatus of claim 7, wherein the preset time is 24 hours.

10. An operating method of a battery management apparatus, the operating method comprising:
communicating with a plurality of sensors (10);
setting identification information of each of the plurality of sensors (10); and
obtaining base line information of each of the plurality of sensors (10)
based on the identification information,
**characterized in that** the plurality of sensors (10) comprises a plurality of gas sensors (10), and
the operating method further comprises:
communicating with a higher-level battery management system, BMS, and
obtaining base line information of each of the plurality of gas sensors (10) from the higher-level battery management system BMS when the plurality of gas sensors (10) are powered off and then on due to an error occurring in the plurality of gas sensors (10).

11. The operating method of claim 10, wherein the plurality of sensors (10) comprises a plurality of gas sensors (10), and
the setting of the identification information of each of the plurality of sensors (10) comprises setting the identification information when the power for identification information allocation is applied to each of the plurality of gas sensors (10).

12. The operating method of claim 10, wherein the plurality of sensors (10) comprises a plurality of gas sensors, and
the setting of the identification information of each of the plurality of sensors (10) comprises applying power for identification information allocation to a first gas sensor (11) connected to the power supply line (30) for identification information allocation among the plurality of gas sensors (10) and setting the identification information.

13. The operating method of claim 12, wherein the setting of the identification information of each of the plurality of sensors (10) comprises setting the identification information when each of the plurality of gas sensors (10) is sequentially applied with the power for identification information allocation from another gas sensor through the power supply line (30) for identification information allocation.

14. The operating method of claim 10, further comprising:
delivering the information obtained from the higher-level battery management system, BMS, to each of the plurality of gas sensors (10) based on the identification information of each of the plurality of gas sensors (10).

## Patentansprüche

1. Batteriemanagement-Vorrichtung (100) umfassend:
eine Kommunikationseinheit (110), welche dazu eingerichtet ist, mit einer Mehrzahl von Sensoren (10) zu kommunizieren; und
eine Steuerung (120), welche dazu eingerichtet ist, Identifikationsinformationen von jedem aus der Mehrzahl von Sensoren (10) festzulegen und Basisinformationen von jedem aus der Mehrzahl von Sensoren (10) auf Grundlage der Identifikationsinformationen zu erhalten,
**dadurch gekennzeichnet, dass** die Mehrzahl von Sensoren (10) eine Mehrzahl von Gassensoren umfasst, und
die Kommunikationseinheit (110) ferner dazu eingerichtet ist, mit einem übergeordneten Batteriemanagement-System, BMS, zu kommunizieren, und
die Steuerung (120) ferner dazu eingerichtet ist, Basisinformationen von jedem aus der Mehrzahl von Gassensoren (10) von dem übergeordneten Batteriemanagement-System, BMS, zu erhalten, wenn die Mehrzahl von Gassensoren (10) abgeschaltet und dann angeschaltet sind, aufgrund eines Fehlers, welcher in der Mehrzahl von Gassensoren (10) auftritt.

2. Batteriemanagement-Vorrichtung nach Anspruch 1, wobei die Mehrzahl von Sensoren (10) eine Mehrzahl von Gassensoren (10) umfasst, und
jeder aus der Mehrzahl von Gassensoren (10) seriell mit einer Leistungsversorgungsleitung (30) für eine Identifikationsinformationen-Allokation verbunden ist.

3. Batteriemanagement-Vorrichtung nach Anspruch 2, wobei die Steuerung (120) ferner dazu eingerichtet ist, die Identifikationsinformationen festzulegen, wenn Leistung für eine Identifikationsinformationen-Allokation an jeden aus der Mehrzahl von Gassensoren (10) angelegt ist.

4. Batteriemanagement-Vorrichtung nach Anspruch 2, wobei die Steuerung (120) ferner dazu eingerichtet ist, Leistung für eine Identifikationsinformationen-Allokation an einen ersten Gassensor (11) anzulegen, welcher mit der Leistungsversorgungsleitung verbunden ist, für eine Identifikationsinformationen-Allokation unter der Mehrzahl von Gassensoren (10), und die Identifikationsinformationen festzulegen.

5. Batteriemanagement-Vorrichtung nach Anspruch 4, wobei die Steuerung (120) ferner dazu eingerichtet ist, die Identifikationsinformationen festzulegen, wenn jeder aus der Mehrzahl von Gassensoren (10) sequenziell mit der Leistung für eine Identifikationsinformationen-Allokation von einem anderen Gassensor (10) durch die Leistungsversorgungsleitung (30) für eine Identifikationsinformationen-Allokation angelegt ist.

6. Batteriemanagement-Vorrichtung nach Anspruch 1, wobei die Steuerung (120) ferner dazu eingerichtet ist, die Informationen, welche von dem übergeordneten Batteriemanagement-System, BMS, erhalten sind, an jeden aus der Mehrzahl von Gassensoren (10) zu liefern, auf Grundlage der Identifikationsinformationen von jedem aus der Mehrzahl von Gassensoren.

7. Batteriemanagement-Vorrichtung nach Anspruch 1, wobei die Steuerung (120) ferner dazu eingerichtet ist, Informationen, welche für eine voreingestellte Zeit durch die Mehrzahl von Sensoren (10) gemessen sind, zu erhalten, wenn ein Fehler in der Mehrzahl von Sensoren (10) nicht auftritt.

8. Batteriemanagement-Vorrichtung nach Anspruch 7, wobei die Kommunikationseinheit (110) ferner dazu eingerichtet ist, mit dem übergeordneten Batteriemanagement-System, BMS, zu kommunizieren, und
die Steuerung (120) ferner dazu eingerichtet ist, die Informationen, welche von der Mehrzahl von Sensoren (10) gemessen sind, zu dem übergeordneten Batteriemanagement-System, BMS, zu liefern.

9. Batteriemanagement-Vorrichtung nach Anspruch 7, wobei die voreingestellte Zeit 24 Stunden ist.

10. Betriebsverfahren einer Batteriemanagement-Vorrichtung, wobei das Betriebsverfahren umfasst:
ein Kommunizieren mit einer Mehrzahl von Sensoren (10);
ein Festlegen von Identifikationsinformationen, von jedem aus der Mehrzahl von Sensoren (10); und
ein Erhalten von Basisinformationen von jedem aus der Mehrzahl von Sensoren (10), auf Grundlage der Identifikationsinformationen,
**dadurch gekennzeichnet, dass** die Mehrzahl von Sensoren (10) eine Mehrzahl von Gassensoren (10) umfasst, und
das Betriebsverfahren ferner umfasst:
ein Kommunizieren mit einem übergeordneten Batteriemanagement-System, BMS; und
ein Erhalten von Basisinformationen von jedem aus der Mehrzahl von Gassensoren (10) von dem übergeordneten Batteriemanagement-System, BMS, wenn die Mehrzahl von Gassensoren (10) abgeschaltet und dann angeschaltet werden, aufgrund eines Fehlers, welcher in der Mehrzahl von Gassensoren (10) auftritt.

11. Betriebsverfahren nach Anspruch 10, wobei die Mehrzahl von Sensoren (10) eine Mehrzahl von Gassensoren (10) umfasst, und
das Festlegen der Identifikationsinformationen von jedem aus der Mehrzahl von Sensoren (10) ein Festlegen der Identifikationsinformationen umfasst, wenn die Leistung für eine Identifikationsinformationen-Allokation an jeden aus der Mehrzahl von Gassensoren (10) angelegt wird.

12. Betriebsverfahren nach Anspruch 10, wobei die Mehrzahl von Sensoren (10) eine Mehrzahl von Gassensoren umfasst, und
das Festlegen der Identifikationsinformationen von jedem aus der Mehrzahl von Sensoren (10) ein Anlegen von Leistung für eine Identifikationsinformationen-Allokation an einen ersten Gassensor (11), welcher mit der Leistungsversorgungsleitung (30) verbunden ist, für eine Identifikationsinformationen-Allokation unter der Mehrzahl von Gassensoren (10) und ein Festlegen der Identifikationsinformationen umfasst.

13. Betriebsverfahren nach Anspruch 12, wobei das Festlegen der Identifikationsinformationen von jedem der Mehrzahl von Sensoren (10) ein Festlegen der Identifikationsinformationen umfasst, wenn jeder aus der Mehrzahl von Gassensoren (10) sequenziell mit der Leistung für eine Identifikationsinformationen-Allokation von einem anderen Gassensor durch die Leistungsversorgungsleitung (30) für eine Identifikationsinformationen-Allokation angelegt wird.

14. Betriebsverfahren nach Anspruch 10, ferner umfassend:
ein Liefern der Informationen, welche von dem übergeordneten Batteriemanagement-System, BMS, erhalten werden, an jeden aus der Mehrzahl von Gassensoren (10), auf Grundlage der Identifikationsinformationen von jedem aus der Mehrzahl von Gassensoren (10).

## Revendications

1. Appareil de gestion de batterie (100) comprenant :
une unité de communication (110) configurée pour communiquer avec une pluralité de capteurs (10) ; et
un dispositif de commande (120) configuré pour définir des informations d'identification de chacun de la pluralité de capteurs (10) et obtenir des informations de référence de chacun de la pluralité de capteurs (10) sur la base des informations d'identification.
**caractérisé en ce que** la pluralité de capteurs (10) comprend une pluralité de capteurs de gaz, et
l'unité de communication (110) est en outre configurée pour communiquer avec un système de gestion de batterie, BMS, de niveau supérieur, et
le dispositif de commande (120) est en outre configuré pour obtenir des informations de référence de chacun de la pluralité de capteurs de gaz (10) à partir du système de gestion de batterie, BMS, de niveau supérieur lorsque la pluralité de capteurs de gaz (10) sont mis hors tension puis sous tension en raison d'une erreur se produisant dans la pluralité de capteurs de gaz (10).

2. Appareil de gestion de batterie selon la revendication 1, dans lequel la pluralité de capteurs (10) comprend une pluralité de capteurs de gaz (10), et
chacun de la pluralité de capteurs de gaz (10) est connecté en série à une ligne d'alimentation électrique (30) pour l'attribution d'informations d'identification.

3. Appareil de gestion de batterie selon la revendication 2, dans lequel le dispositif de commande (120) est en outre configuré pour définir les informations d'identification lorsqu'une alimentation pour l'attribution d'informations d'identification est appliquée à chacun de la pluralité de capteurs de gaz (10).

4. Appareil de gestion de batterie selon la revendication 2, dans lequel le dispositif de commande (120) est en outre configuré pour appliquer une alimentation pour l'attribution d'informations d'identification à un premier capteur de gaz (11) connecté à la ligne d'alimentation électrique pour l'attribution d'informations d'identification parmi la pluralité de capteurs de gaz (10) et définir les informations d'identification.

5. Appareil de gestion de batterie selon la revendication 4, dans lequel le dispositif de commande (120) est en outre configuré pour définir les informations d'identification lorsque chacun de la pluralité de capteurs de gaz (10) est appliqué séquentiellement avec l'alimentation pour l'attribution d'informations d'identification d'un autre capteur de gaz (10) à travers la ligne d'alimentation électrique (30) pour l'attribution d'informations d'identification.

6. Appareil de gestion de batterie selon la revendication 1, dans lequel le dispositif de commande (120) est en outre configuré pour fournir les informations obtenues à partir du système de gestion de batterie, BMS, de niveau supérieur à chacun de la pluralité de capteurs de gaz (10) sur la base des informations d'identification de chacun de la pluralité de capteurs de gaz.

7. Appareil de gestion de batterie selon la revendication 1, dans lequel le dispositif de commande (120) est en outre configuré pour obtenir des informations mesurées pendant un temps prédéfini par la pluralité de capteurs (10) lorsqu'une erreur ne se produit pas dans la pluralité de capteurs (10).

8. Appareil de gestion de batterie selon la revendication 7, dans lequel l'unité de communication (110) est en outre configurée pour communiquer avec le système de gestion de batterie, BMS, de niveau supérieur, et
le dispositif de commande (120) est en outre configuré pour fournir les informations mesurées à partir de la pluralité de capteurs (10) au système de gestion de batterie, BMS, de niveau supérieur.

9. Appareil de gestion de batterie selon la revendication 7, dans lequel le temps prédéfini est de 24 heures.

10. Procédé de fonctionnement d'un appareil de gestion de batterie, le procédé de fonctionnement comprenant :
la communication avec une pluralité de capteurs (10) ;
la définition d'informations d'identification de chacun de la pluralité de capteurs (10) ;
et l'obtention d'informations de référence de chacun de la pluralité de capteurs (10) sur la base des informations d'identification,
**caractérisé en ce que** la pluralité de capteurs (10) comprend une pluralité de capteurs de gaz (10), et
le procédé de fonctionnement comprend en outre :
la communication avec un système de gestion de batterie, BMS, de niveau supérieur ; et
l'obtention d'informations de référence de chacun de la pluralité de capteurs de gaz (10) à partir du système de gestion de batterie, BMS, de niveau supérieur lorsque la pluralité de capteurs de gaz (10) sont mis hors tension puis sous tension en raison d'une erreur se produisant dans la pluralité de capteurs de gaz (10).

11. Procédé de fonctionnement selon la revendication 10, dans lequel la pluralité de capteurs (10) comprend une pluralité de capteurs de gaz (10), et
la définition des informations d'identification de chacun de la pluralité de capteurs (10) comprend la définition des informations d'identification lorsque l'alimentation pour l'attribution d'informations d'identification est appliquée à chacun de la pluralité de capteurs de gaz (10).

12. Procédé de fonctionnement selon la revendication 10, dans lequel la pluralité de capteurs (10) comprend une pluralité de capteurs de gaz, et
la définition des informations d'identification de chacun de la pluralité de capteurs (10) comprend l'application d'une alimentation pour l'attribution d'informations d'identification à un premier capteur de gaz (11) connecté à la ligne d'alimentation électrique (30) pour l'attribution d'informations d'identification parmi la pluralité de capteurs de gaz (10) et la définition des informations d'identification.

13. Procédé de fonctionnement selon la revendication 12, dans lequel la définition des informations d'identification de chacun de la pluralité de capteurs (10) comprend la définition des informations d'identification lorsque chacun de la pluralité de capteurs de gaz (10) est appliqué séquentiellement avec l'alimentation pour l'attribution d'informations d'identification d'un autre capteur de gaz à travers la ligne d'alimentation électrique (30) pour l'attribution d'informations d'identification.

14. Procédé selon la revendication 10, comprenant en outre :
la fourniture des informations obtenues à partir du système de gestion de batterie, BMS, de niveau supérieur à chacun de la pluralité de capteurs de gaz (10) sur la base des informations d'identification de chacun de la pluralité de capteurs de gaz (10).
